# EUROPEAN PATENT APPLICATION

(11) **EP 4 539 061 A1**
(43) Date of publication of application: **16.04.2025**
(21) Application number: 23824207.7
(22) Date of filing: 13.06.2023
(51) Int. Cl.: G16H 50/20, G16H 15/00, G16H 10/60, A61B 5/0245, G06F 3/0482

(54) **APPARATUS AND METHOD FOR EXTRACTING MEDICAL INFORMATION**

(30) Priority: 13.06.2022 KR 20220071586
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2023/008120
(87) International publication number: WO 2023/243992

(57) **Abstract**

Disclosed are an apparatus and method for extracting medical information, according to an embodiment. The apparatus for extracting medical information, according to an embodiment, comprises: one or more processors; and a memory that stores instructions for executing the one or more processors, wherein the processors: detect a user input requesting extraction of medical information of a target image, which includes attribute data of the target image and feature data of the target image; extract the attribute data of the target image in response to the user input; extract the medical information by classifying the feature data of the target image; and specify, by using a pre-trained model, a region of interest within the target image, which corresponds to the target image and the medical information.

## Description

### Technical Field

The disclosed embodiments relate to an apparatus and method for extracting medical information.

### Cross-References to Related Applications

This application claims priority to Korean Provisional Application No. 10-2022-0071586, filed on Jun. 13, 2022, the entire contents of which are incorporated into this application.

### Background

In general, medical information is managed through a Picture Archiving and Communication System (PACS). However, PACS is in principle limited in processing for reasons of patient privacy.

Even if processing is possible, the uploading and downloading of medical information takes place over several steps and involves cumbersome procedures. Moreover, for privacy reasons, the progress of those procedures also requires the patient's consent.

Furthermore, the medical information managed in PACS originates from various medical devices and its formats and protocols differ for each medical device. That is, in a case where medical devices differ, compatibility between medical information is considerably insufficient.

Therefore, in order to enhance the patient privacy and the ease of processing medical information, there is a need for a system that integrally executes a series of medical information extraction processes with only one execution.

### Detailed Description

### Technical Problems

The disclosed embodiments are for executing a series of multiple steps of extracting medical information in one queue in succession with one command.

### Technical Solution

An apparatus for extracting medical information according to an embodiment includes: one or more processors; and a memory storing instructions for executing the one or more processor, wherein the processor: detects a user input requesting extraction of medical information for a target image including attribute data of the target image and feature data of the target images, extracts the attribute data of the targets image in response to the user input, classifies the feature data of the targets images to extract the medical information, and specifies a region of interest in the target image corresponding to the target image and the medical information by using a pre-trained model.

The attribute data may include at least one of a resolution and a number of channels of the target image, and the feature data may include classification prediction values for at least one of a modality of the target image, a photographing direction and a number of signal channels.

The processors may detect, by using a pre-trained model, one or more candidate bounding boxes corresponding to each of one or more objects included in the target image, calculate a confidence score corresponding to each of the one or more candidate bounding boxes, and sort the one or more candidates bounding boxes based on the confidence score.

The processors may remove overlapping bounding boxes based on the confidence score of each of the one or more candidate bounding boxes, and specify the region of interest based on at least one of the remaining one or more candidate bounding boxes.

The processors may select a reference bounding box based on a confidence score of each of the one or more candidate bounding boxes, remove overlapping bounding boxes in the one or more candidates bounding boxes based on a matching rate between a prediction region of the reference bounding box and a prediction region of a remaining to-be-compared bounding box, and specify the region of interest based on a candidate bounding box having the highest confidence score among the remaining one or more candidate bounding boxes.

The processors, when detecting a user input comprising at least one of a pre-defined mouse gesture input, a shortcut key input, an icon input and a voice command, may initiate extraction of the medical information using a pre-trained model.

The processors may specify a boundary of the region of interest based on a second user input specifying the region of interest in the target image, and manually extract the medical information based on a third user input specifying an attribute of the region of interest.

The processors may output an device list comprising one or more medical devices in communication with the apparatus for extracting medical information, and send at least one of the target image, the region of interest, the attribute data and the feature data to medical device selected based on a fourth user input in the device list.

The processors, when receiving a fifth user input of dropping the target image or the region of interest into a pre-set region, may transmit it to a model, among the one or more pre-trained models, that matches the target image based on at least one of the attribute data and the feature data, cause the matching model to perform medical analysis on the target image, and upon completion of the medical analysis, output at least one of a visual notification signal and an auditory notification signal.

The processors may output a warning signal when at least one of a vertical resolution, a horizontal resolution, and a ratio between the vertical resolution and the horizontal resolution of the target image is less than or equal to a pre-set value.

A method for extracting medical information, according to an embodiment, is a method performed by an apparatus for extracting medical information, comprising: one or more processors; and a memory storing instructions for executing the one or more processors, the method may include detecting a user input requesting extraction of medical information of a target image comprising attribute data of the target image and feature data of the target images; extracting attribute data of the target image in response to the user input, and extracting the medical information by classifying the feature data of the target image; and specifying, by using a pre-trained model, a region of interest within the target image, which corresponds to the target image and the medical information.

The attribute data may include at least one of a resolution and a number of channels of the target image, and the feature data may include classification prediction values for at least one of a modality of the target image, a photographing direction and a number of signal channels.

The extracting of the medical information may include detecting, by using a pre-trained model, one or more candidate bounding boxes corresponding to each of one or more objects included in the target image; calculating a confidence score corresponding to each of the one or more candidate bounding boxes; and sorting the one or more candidates bounding boxes based on the confidence score.

The extracting of the medical information may include removing overlapping bounding boxes based on the confidence score of each of the one or more candidate bounding boxes; and specifying the region of interest based on at least one of the remaining one or more candidate bounding boxes.

The extracting of the medical information may include select a reference bounding box based on a confidence score of each of the one or more candidate bounding boxes; removing overlapping bounding boxes in the one or more candidates bounding boxes based on a matching rate between a prediction region of the reference bounding box and a prediction region of a remaining to-be-compared bounding box; and specifying the region of interest based on the remaining one or more candidate bounding boxes.

The extracting of the medical information may include, when detecting a user input comprising at least one of a pre-defined mouse gesture input, a shortcut key input, an icon input and a voice command, initiating extraction of the medical information using a pre-trained model.

The extracting of the medical information may include specifying a boundary of the region of interest based on a second user input specifying the region of interest in the target image; and manually extracting the medical information based on a third user input specifying an attribute of the region of interest.

The method may include outputting an device list comprising one or more medical devices in communication with the apparatus for extracting medical information; and sending at least one of the target image, the region of interest, the attribute data and the feature data to medical device selected based on a fourth user input in the device list.

The sending may include, when receiving a fifth user input of dropping the target image or the region of interest into a pre-set region, transmitting it to a model, among the one or more pre-trained models, that matches the target image based on at least one of the attribute data and the feature data; causing the matching model to perform medical analysis on the target image; and upon completion of the medical analysis, outputting at least one of a visual notification signal and an auditory notification signal.

The method may further include outputting a warning signal when at least one of a vertical resolution, a horizontal resolution, and a ratio between the vertical resolution and the horizontal resolution of the target image is less than or equal to a pre-set value.

### Advantageous Effects

The disclosed embodiments may easily execute a series of multiple steps of extracting medical information in one queue in succession with one command.

### Brief Description of the Drawings

FIG. 1 is a block diagram for illustrating an apparatus for extracting medical information according to an embodiment.
FIG. 2 is a diagram of an example for illustrating menu and icon for invoking an extraction function of an apparatus for extracting medical information.
FIG. 3 is a diagram of an example for illustrating menu, icon, and sub-menus for invoking an extraction function of an apparatus for extracting medical information.
FIG. 4 is a diagram of an example for illustrating a process of extracting medical information by an apparatus for extracting medical information.
FIG. 5 is a diagram of an example for illustrating a process in which an apparatus for extracting medical information uploads a target image.
FIG. 6 is a diagram of an example of a screen where an apparatus for extracting medical information provides extracted medical information to a user.
FIG. 7 is a diagram of another example of a screen where an apparatus for extracting medical information provides extracted medical information to a user.
FIG. 8 is a flowchart for describing a method of extracting medical information according to an embodiment.

### Embodiments of the Invention

Hereinafter, a specific embodiment of an example will be described with reference to the drawings. The detailed description below is provided to facilitate a comprehensive understanding of the disclosure described herein. However, this is merely an example, and the present disclosure is not limited thereto.

In describing the embodiments, detailed descriptions of known technologies related to the present disclosure will be omitted if it is determined that they could unnecessarily obscure the gist of the embodiment.

The terms described below are terms defined in consideration of the work in the present disclosure, which may vary according to the intention or practice of a user, an operator, or the like. Therefore, the definition should be based on the contents throughout the specification. The terminology used in the detailed description is for the purpose of describing one embodiment only and should in no way be limiting. Unless expressly specified otherwise, the expressions in singular form include meanings in plural form. In the present description, expressions such as "comprising" or "including" are intended to refer to certain components, numbers, steps, operations, elements, parts or combinations thereof, and should not be interpreted to exclude the presence or possibility of one or more other components, numbers, steps, operations, elements, parts or combinations thereof other than those described.

Furthermore, embodiments described herein may have aspects that are wholly in hardware, partly in hardware and partly in software, or wholly in software. In this specification, a "unit", a "layer", a "module", a "device", a "server", a "system", or the like refers to a computer-related entity such as hardware, a combination of hardware and software, or software. For example, a unit, a layer, a module, a device, a server, or a system may refer to hardware constituting a part or all of a platform and/or software such as an application for driving the hardware. As a specific example, a unit, a layer, a module, a device, a server, or a system may be implemented by a processor.

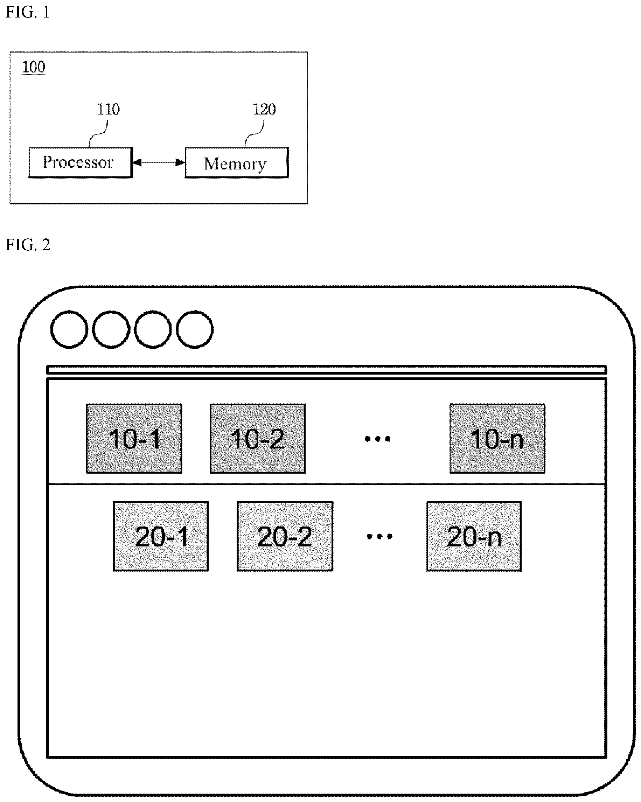

FIG. 1 is a block diagram for illustrating an apparatus 100 for extracting medical information according to an embodiment.

The apparatus 100 for extracting medical information according to an embodiment is a device that provides an easy data-pipe line to an obtained target image. Specifically, the apparatus 100 for extracting medical information may refer to an apparatus that detects a user input and extracts medical information in a queue.

Here, the medical information may include the region of interest selected as appropriate to be captured or cropped in the target image, the type of the target image, its format, etc.

Referring to FIG. 1, an apparatus 100 for extracting medical information includes a processor 110 and a memory 120.

The processor 110 detects a user input requesting extraction of medical information of the target image, which includes attribute data of the target image and feature data of the target image.

Here, the target image may be at least one of the images that include the patient's bio-signal and the results of medical analysis analyzing the bio-signal. For example, the target image may include at least one of an electrocardiogram image and a chest radiographic image.

When detecting a pre-defined user input, the processor 110 may initiate extraction of the medical information using a pre-trained model.

Here, the pre-defined user input may include, for example, at least one of a mouse gesture input, a shortcut key input, a menu click input, an icon click input, and a voice command, mapped to initiate extraction of medical information.

As a specific example, when detecting a mouse click input on the target image, the processor 110 may continuously perform a series of steps necessary for extracting medical information of the target image and specifying the region of interest.

As a specific example, when detecting of a shortcut key input mapped to the predicted class corresponding to the target image, the processor 110 may continuously perform the steps of specifying region of interest corresponding to the shortcut key.

That is, the processor 110 may be interpreted as invoking the extraction function when detecting a user input while waiting for initiation of the extraction function in the background.

In this case, the processor 110 may change the visual signal, including the shape, appearance, color, and/or brightness, etc. of the screen or user interface, to make the user aware that the user input has been detected and requested.

On the other hand, although the user input that initiates extraction of medical information has been described as being a mouse input, a shortcut key input, or the like, this is illustrative and is not necessarily limited to the listed examples.

The processor 110 extracts attribute data of a target image in response to a user input, and extracts the medical information by classifying the feature data of the target image.

The attribute data is data for describing a property of the target image, and may include, for example, at least one of a resolution, a number of channels, a color space, a bit depth, an image format, a contamination level, a noise level, and a compression method of the target image. In this case, the attribute data may be expressed in an array format.

The feature data may be data including at least one of a modality of the target image, a photographing direction, and a number of signal channels. In this case, the feature data may be expressed as a set of classes predicted by the pre-trained model.

Specifically, the feature data may include a prediction value that classifies what type of medical image the target image is. As a specific example, the feature data may be expressed as a corresponding class by classifying the modality into electrocardiogram or chest radiograph.

As another example, the feature data may be expressed as a corresponding class by classifying the photographing direction of the target image into a posteroanterior direction (PA), an anteroposterior direction (AP), or a lateral direction (Lat). As another example, when the feature data is signal data such as an electrocardiogram, at least one of the total number of channels and the types of channels constituting the signal data may be predicted and expressed as a corresponding class. For example, the feature data may be expressed as a corresponding class by predicting the number of signal channels of the electrode used for inspection corresponding to the target image in 1 channel (e.g., lead II), 3 channels (e.g, lead I, II, III), and 12 channels.

The processor 110 specifies, by using a pre-trained model, a region of interest within the target image, which corresponds to the target image and the medical information.

According to an example, the processor 110 may specify the region of interest within the target image by combining attribute data and feature data using a pre-trained model.

Here, the region of interest may refer to a region within the target image that affects the result of the medical diagnosis. In other words, the region of interest is a region in which important objects that influence the medical diagnosis result are included, and a plurality of regions may exist within a single target image.

According to an example, the processor 110 may detect, by using a pre-trained model, one or more candidate bounding boxes corresponding to the objects included in the target image to specify the region of interest.

According to an example, the processor 110 may specify, as a region of interest, at least one of the one or more candidate bounding boxes based on a confidence score of the detected one or more candidate bounding boxes in the target image.

Here, the confidence score may be a probability of a bounding box defining a region including at least one of an electrocardiogram image and a chest radiographic image in a predetermined manner in the trained model and a statistical definition corresponding thereto, and may include an image detail type and a region of the electrocardiogram, and a detail type and a regions of the chest radiographic image.

Specifically, the processor 110 may define a class and a region for the detected one or more candidate bounding boxes, and specify at least one of the candidate bounding boxes as the region of interest, based on a confidence score of each bounding box.

The processor 110 may select a bounding box having the highest confidence score as a predicted correct answer, and calculate a matching rate between a prediction region of another one or more candidate bounding boxes and the selected correct answer value, to remove overlapping bounding boxes in the one or more candidate bounding boxes.

The processor 110 may then select the bounding box having the highest confidence score among the remaining bounding boxes in the same manner, and repeat the process of removing overlapping bounding boxes to specify the region of interest and the corresponding class for the target image.

The processor 110 may apply at least one of the attribute data and the specific data to a pre-defined candidate extraction function to select any bounding box as the region of interest. Alternatively, the processor 110 may specify the candidate bounding box selected by the user, as the region of interest. According to an example, the processor 110 may also manually detect the region of interest by user input.

Specifically, the processor 110 may specify a boundary and a class of the region of interest based on the second user input of specifying the region of interest in the target image.

For example, the processor 110 may manually specify the region of interest based on at least one of the drag input and the click input when detecting a second input of a second user of the user that includes at least one of a drag input and a click input that specifies a boundary of the region of interest. A new window may be displayed to allow the user to specify a class before, during, or after the drag input and click start, or the user may be made aware that a class can be specified, including by changing at least one of the shape, appearance, color, and brightness of the bounding box. Additionally, the class can be specified through additional input.

The processor 110 may determine the extraction suitability of the target image before specifying the region of interest. In other words, the processor 110 may determine whether the target image is suitable to be used as an extraction target of the medical information.

Specifically, the processor 110 may determine whether the target image meets a pre-set qualitative or morphological condition by using attribute data of the target image, to determine extraction suitability of the target image.

For example, the processor 110 may exclude the target image from the extraction target when at least one of the vertical resolution, the horizontal resolution, and the ratio between the vertical resolution and the horizontal resolution of the target image is less than or equal to a pre-set value.

The processor 110 may determine whether the target image meets a pre-set qualitative condition by using the attribute data, and determine extraction suitability of the target image.

For example, the processor 110 may exclude the target image from the extraction target when the contamination level and the noise level of the target image are greater than or equal to pre-set values. When the image format of the target image does not conform to the pre-defined format, the processor 110 may exclude the target image from the extraction target.

When the processor 110 determines that the target image does not satisfy at least one of the morphological condition and the qualitative condition for being used as the target image, it may output a warning signal.

The processor 110 may qualitatively and/or quantitatively evaluate the extraction suitability based on at least one of the morphological condition result and the qualitative condition result of the target image.

The memory 120 stores one or more instructions that the processor 110 executes.

The memory 120 may store various data used by the processor 110. For example, the memory 120 may include software (e.g., a program executed by the processor 110 and/or instructions related to the program, a library related to the instructions, input data or output data for the instructions).

The memory 120 may include a volatile memory 120 or a nonvolatile memory 120.

FIG. 2 is a diagram of an example for illustrating menu 10 and icon 20 for invoking an extraction function of the apparatus 100 for extracting medical information.

Referring to FIG. 2, the first image includes menu 10 and icon 20.

The processor 110 may display the menu 10 on top of the first image. The processor 110 may aggregate and display one or more menus 10 on a toolbar to improve user convenience.

The processor 110 may display the icon 20 in the content region of the first image. The content region is a lower region below the toolbar and may include a region in which a user performs an operation. For example, it may be a region in which an operation of editing an image, uploading an image, or the like is performed.

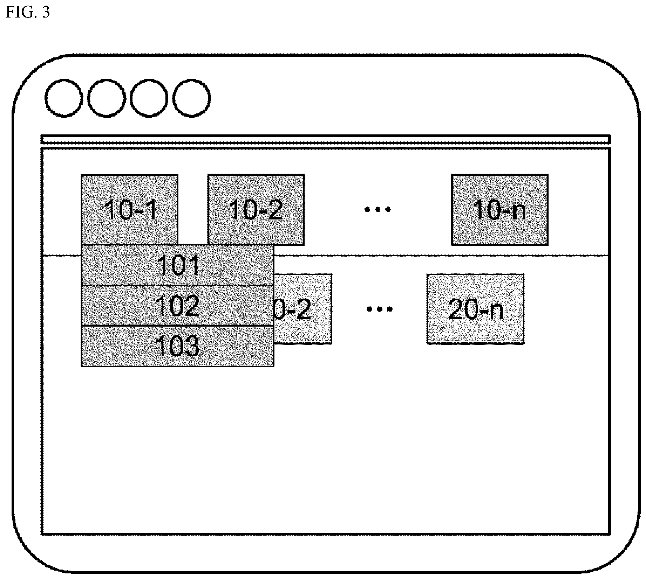

FIG. 3 is a diagram of an example for illustrating menu 10, icon 20, and sub-menus 101, 102, 103 for invoking an extraction function of the apparatus 100 for extracting medical information.

Referring to FIG. 3, the second image displays menu 10 and sub-menus 101, 102, 103. Specifically, the processor 110 outputs one or more sub-menus 101, 102, 103 that are displayed upon selection of the menu 10 based on the user input.

In this case, the processor 110 may change at least one of the shape, appearance, color, and brightness of the selected (e.g., clicked) menu 10 through a user input so that the user may recognize that the selected menu 10 has been executed.

Thereafter, the processor 110 may output the sub-menu 101, 102, 103 included in the selected menu 10 in the vicinity of the selected menu 10 so as to enable the user to execute the operations of the sub-menus 101, 102, 103. For example, the processor 110 may output the sub-menus 101, 102, 103 in the form of unfolding the sub-menus 101, 102, 103 at the bottom of the menu 10.

That is, the processor 110 may overlap and output the sub-menus 101, 102, 103 of the menu 10 in the same image from which the menu 10 is output. For example, when the menu 10 is disposed in the first image, the processor 110 may output the sub-menus 101, 102, 103 in the first image. When the menu 10 is disposed in the second image, the processor 110 may output the sub-menus 101, 102, 103 in the second image.

In this case, at least one of the menu 10 and the sub-menus 101, 102, 103 may be visualized via at least one of intuitive symbols, appearances, and images describing the corresponding operation.

For example, the menu 10 or sub-menus 101, 102, 103 associated with a capture operation may be visualized as an image including a logo, mark, symbol, etc. that is shaped like a camera, thereby intuitively providing a description of the capture operation.

In another example, the menu 10 associated with an upload may be visualized as an image including a logo, mark, symbol, etc. that is shaped like an upward arrow, thereby intuitively providing a description of the upload operation.

On the other hand, when a user input requesting execution of the icon 20 is received, the processor 110 may directly execute the sub-menus 101, 102, 103 corresponding to the icon 20 without querying one or more sub-menus 101, 102, 103 included in the menu 10.

The icon 20 may be visualized through at least one of intuitive symbols, appearances, and images that describe corresponding operation.

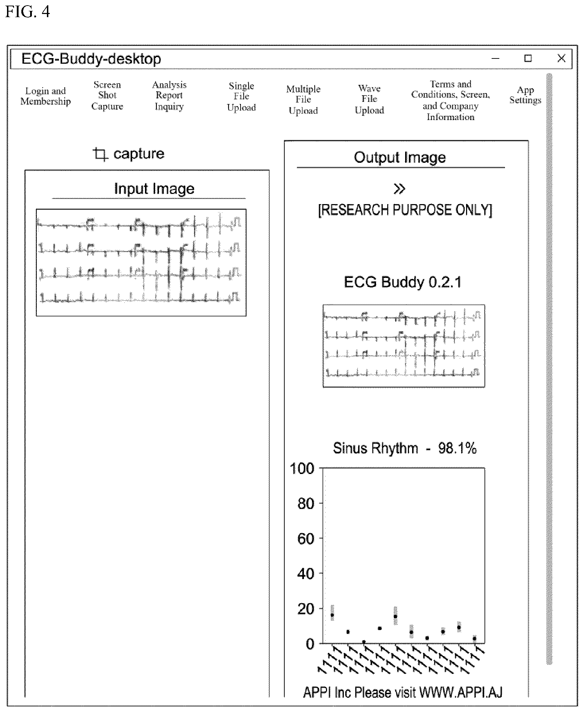

FIG. 4 is a diagram of an example for illustrating a process of extracting medical information by the apparatus 100 for extracting medical information.

The target image in FIG. 4 is selected as the target image, and is in a state of waiting for invocation of the extraction function according to the user input.

Here, the input image is a medical image (e.g., an electrocardiogram image) obtained by the apparatus 100 that extracts medical information as a target of medical analysis, and may be, for example, an image obtained from an electronic medical record.

When detecting the pre-defined user input, the processor 110 may extract medical information of the target image to be uploaded or displayed on the screen.

Here, the processor 110 may specify the region of interest by using a pre-trained model in an automatic designation manner.

The processor 110 may specify the region of interest in the target image by using a trained model mapped to the input shortcut key. For example, when the shortcut key "1" mapped to be equal to "1" designated as the class of the electrocardiogram image is input, the processor 110 may specify the region of interest customized in the electrocardiogram image by using the pre-trained model.

The processor 110 may send the region of interest to an analysis model that is mapped based on at least one of the attribute data, the feature data, and the user input, and cause the analysis model to analyze the medical diagnosis corresponding to the region of interest.

In another example, the processor 110 may specify the region of interest based on the user input, in the manner of a manual designation.

Specifically, the processor 110 may specify the region of interest based on a user's drag input in a diagonal direction. Alternatively, the processor 110 may specify the region of interest based on a user's click or touch input selecting the coordinates.

As a specific example, the processor 110 may manually specify the region of interest via a user input that drags from a start point to an end point. The processor 110 may manually specify the region of interest based on user input that click or touch the four points.

The processor 110 may generate a captured image based on the region of interest after the region of interest is specified. Then, the processor 110 may display, on the same screen, a list of medical devices that may transmit the captured image after generating the captured image.

Specifically, when detecting a user's right-click input or a click input of the icon 20 that executes a corresponding operation, the processor 110 may display, on the same screen, a list of medical devices that may be automatically transmitted.

Thereafter, when receiving the user input of dropping the captured image on the pre-set region, the processor 110 may cause the analysis model to perform medical analysis corresponding to the type of bio-signal of the target image.

Then, upon completion of the medical analysis, the processor 110 may cause the apparatus 100 for extracting medical information to output at least one of a visual notification signal and an auditory notification signal.

For example, the processor 110 may cause the apparatus 100 for extracting medical information to output a notification signal including at least one of a pop-up message, an animation effect, and a notification sound to the display.

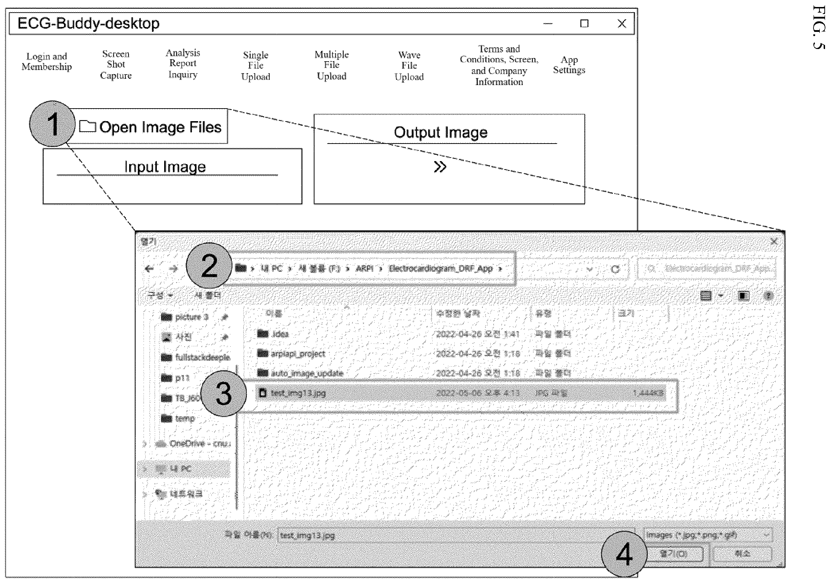

FIG. 5 is a diagram of an example for illustrating a process in which an apparatus 100 for extracting medical information uploads a target image.

When receiving a user input for selecting the second sub-menu 102 or the second icon 20-2, the processor 110 may execute a file search to display a storage path of the target image.

Thereafter, when receiving the user input selecting the target image, the processor 110 may retrieve the selected target image. Here, the target image may be an image previously stored in the apparatus 100 for extracting medical information or a medical image that may be provided from the outside.

After uploading the image, the processor 110 may automatically perform designated operation necessary for extracting medical information without user input. For example, the processor 110 may extract medical information and request medical analysis, without user input, after uploading the target image.

After executing a specific operation automatically or manually, the processor 110 may display a specific icon 20 on the screen. For example, a specific icon 20 may be an icon 20 that executes an operation that is most frequently used after the specific operation. As a specific example, after medical information extraction, if there are frequent requests from the user to store the region of interest, the processor 110 may display, on the screen, the icon 20 requesting storage of the region of interest after medical information extraction.

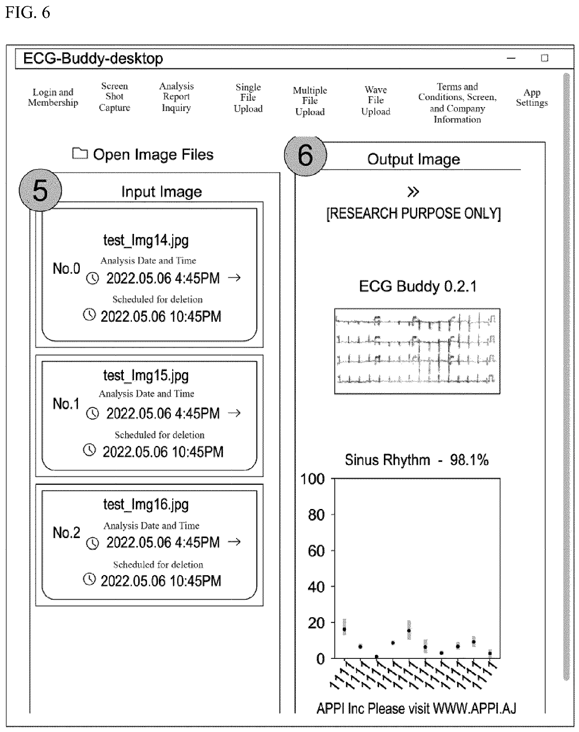

FIG. 6 is a diagram of an example of a screen where an apparatus 100 for extracting medical information provides extracted medical information to a user.

The processor 110 may display the requested medical information on the screen to provide the medical information requested by the user.

Specifically, the processor 110 may display information related to the target image on the screen. For example, the information related to the target image may include a file name, an upload time, a query request order etc., of the image.

Specifically, the processor 110 may display a result of the medical analysis of the target image on the screen. In this case, the result of the medical analysis may refer to a result of a medical analysis analyzed based on medical information by using a pre-trained model. Here, the result of the medical analysis may include at least one of a patient name, a patient number identification code, and a diagnosis name.

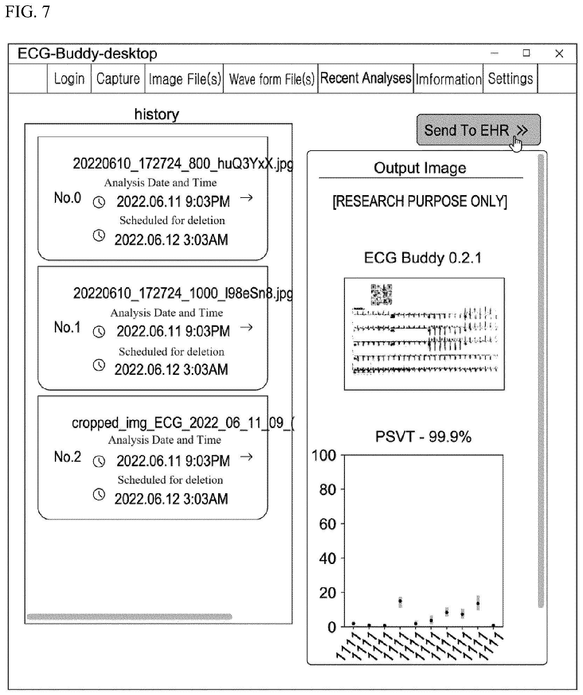

FIG. 7 is a diagram of another example of a screen where an apparatus 100 for extracting medical information provides extracted medical information to a user.

The processor 110 may send at least one of information related to the target image and a result of the medical analysis to a server of the medical institution.

At this time, the processor 110 may transmit only the sending request of the pre-authenticated user to the server of the medical institution without the user's consent.

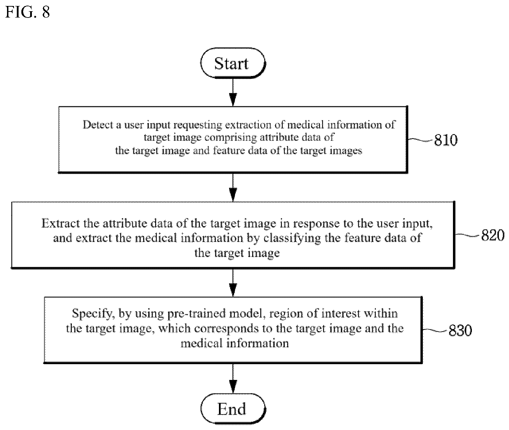

FIG. 8 is a flowchart for illustrating a method of providing a medical platform according to an embodiment.

Referring to FIG. 8, it may be performed by the apparatus 100 for extracting medical information according to an embodiment of FIG. 1.

First, the apparatus 100 for extracting medical information according to an embodiment detects 810 a user input requesting extraction of medical information including attribute data of the target image and feature data of the target image from the target image.

Then, the apparatus 100 for extracting medical information according to an embodiment extracts 820 attribute data of the target image in response to the user input, and extracts the medical information by classifying the feature data of the target image.

Then, the apparatus 100 for extracting medical information according to an embodiment specifies 830, by using a pre-trained model, a region of interest within the target image, which corresponds to the target image and the medical information.

On the other hand, an embodiment of the present disclosure may include a program for performing the methods described in the present specification on a computer, and a computer-readable recording medium including the program. The computer-readable recording medium may include program command, local data files, local data structures, and the like, alone or in combination. The media may be those specially designed and constructed for the present disclosure, or those commonly used in the field of computer software. Examples of the computer-readable recording medium include magnetic media such as a hard disk, a floppy disk, and a magnetic tape, optical recording media such as a CD-ROM and a DVD, and hardware devices specifically configured to store and perform program command, such as a ROM, a RAM, and a flash memory 120. Examples of the program may include machine language codes such as those made by a compiler, as well as high-level language codes that may be executed by a computer using an interpreter or the like.

While exemplary embodiments of the present disclosure have been described in detail above, those skilled in the art will appreciate that various modifications may be made to the above-described embodiments without departing from the scope of the present disclosure. Therefore, the scope of the present disclosure should not be limited to the described embodiments, but should be defined not only by the claims described below but also by their equivalents.

### Industrial Availability

An apparatus and method for extracting medical information according to an embodiment are available to the digital medical industry by executing a series of medical information extraction processes in one queue with one user command.

## Claims

1. A apparatus for extracting medical information, comprising:
one or more processors; and
a memory storing instructions for executing the one or more processors,
wherein the processors:
detect a user input requesting extraction of medical information of a target image, which comprises attribute data of the target image and feature data of the target image;
extract the attribute data of the target image in response to the user input;
extract the medical information by classifying the feature data of the target image; and
specify, by using a pre-trained model, a region of interest within the target image, which corresponds to the target image and the medical information.

2. The apparatus for extracting medical information of claim 1, wherein
the attribute data comprises at least one of a resolution and a number of channels of the target image; and
the feature data comprises classification prediction values for at least one of a modality, a photographing direction and a number of signal channels of the target image.

3. The apparatus for extracting medical information of claim 1, wherein
the processors:
detect, by using a pre-trained model, one or more candidate bounding boxes corresponding to each of one or more objects comprised in the target image;
calculate a confidence score corresponding to each of the one or more candidate bounding boxes;
and
sort the one or more candidates bounding boxes based on the confidence score.

4. The apparatus for extracting medical information of claim 3, wherein
the processors:
remove overlapping bounding boxes based on the confidence score of each of the one or more candidate bounding boxes; and
specify the region of interest based on at least one of the remaining one or more candidate bounding boxes.

5. The apparatus for extracting medical information of claim 4, wherein
the processors:
select a reference bounding box based on a confidence score of each of the one or more candidate bounding boxes;
remove overlapping bounding boxes in the one or more candidates bounding boxes based on a matching rate between a prediction region of the reference bounding box and a prediction region of a remaining to-be-compared bounding box; and
specify the region of interest based on a candidate bounding box having the highest confidence score among the remaining one or more candidate bounding boxes.

6. The apparatus for extracting medical information of claim 1, wherein
the processors:
when detecting a user input comprising at least one of a pre-defined mouse gesture input, a shortcut key input, an icon input and a voice command, initiate extraction of the medical information using a pre-trained model.

7. The apparatus for extracting medical information of claim 1, wherein
the processors:
specify a boundary of the region of interest based on a second user input specifying the region of interest in the target image; and
manually extract the medical information based on a third user input specifying an attribute of the region of interest.

8. The apparatus for extracting medical information of claim 1, wherein
the processors:
output an device list comprising one or more medical devices in communication with the apparatus for extracting medical information; and
send at least one of the target image, the region of interest, the attribute data and the feature data to medical device selected based on a fourth user input in the device list.

9. The apparatus for extracting medical information of claim 8, wherein
the processors:
when receiving a fifth user input of dropping the target image or the region of interest into a pre-set region, transmit it to a model, among the one or more pre-trained models, that matches the target image based on at least one of the attribute data and the feature data;
cause the matching model target image; and
upon completion of the medical analysis, output at least one of a visual notification signal and an auditory notification signal.

10. The apparatus for extracting medical information of claim 1, wherein
the processors:
output a warning signal when at least one of a vertical resolution, a horizontal resolution, and a ratio between the vertical resolution and the horizontal resolution of the target image is less than or equal to a pre-set value.

11. A method for extracting medical information, performed by an apparatus for extracting medical information comprising:
one or more processors; and
a memory storing instructions for executing the one or more processors,
the method comprising:
detecting a user input requesting extraction of medical information of a target image comprising attribute data of the target image and feature data of the target images;
extracting attribute data of the target image in response to the user input, and extracting the medical information by classifying the feature data of the target image; and
specifying, by using a pre-trained model, a region of interest within the target image, which corresponds to the target image and the medical information.

12. The method for extracting medical information of claim 11, wherein
the attribute data comprises at least one of a resolution and a number of channels of the target image; and
the feature data comprises classification prediction values for at least one of a modality of the target image, a photographing direction and a number of signal channels.

13. The method for extracting medical information of claim 11, wherein
the extracting of the medical information comprises:
detecting, by using a pre-trained model, one or more candidate bounding boxes corresponding to each of one or more objects comprised in the target image;
calculating a confidence score corresponding to each of the one or more candidate bounding boxes; and
sorting the one or more candidates bounding boxes based on the confidence score.

14. The method for extracting medical information of claim 13, wherein
the extracting of the medical information comprises:
removing overlapping bounding boxes based on the confidence score of each of the one or more candidate bounding boxes; and
specifying the region of interest based on at least one of the remaining one or more candidate bounding boxes.

15. The method for extracting medical information of claim 14, wherein
the extracting of the medical information comprises:
select a reference bounding box based on a confidence score of each of the one or more candidate bounding boxes;
removing overlapping bounding boxes in the one or more candidates bounding boxes based on a matching rate between a prediction region of the reference bounding box and a prediction region of a remaining to-be-compared bounding box; and
specifying the region of interest based on the remaining one or more candidate bounding boxes.

16. The method for extracting medical information of claim 11, wherein
the extracting of the medical information comprises:
when detecting a user input comprising at least one of a pre-defined mouse gesture input, a shortcut key input, an icon input and a voice command, initiating extraction of the medical information using a pre-trained model.

17. The method for extracting medical information of claim 11, wherein
the extracting of the medical information comprises:
specifying a boundary of the region of interest based on a second user input specifying the region of interest in the target image; and
manually extracting the medical information based on a third user input specifying an attribute of the region of interest.

18. The method for extracting medical information of claim 11, wherein
the method comprises:
outputting an device list comprising one or more medical devices in communication with the apparatus for extracting medical information; and
sending at least one of the target image, the region of interest, the attribute data and the feature data to medical device selected based on a fourth user input in the device list.

19. The method for extracting medical information of claim 18, wherein
the sending comprises:
when receiving a fifth user input of dropping the target image or the region of interest into a pre-set region, transmitting it to a model, among the one or more pre-trained models, that matches the target image based on at least one of the attribute data and the feature data;
causing the matching model to perform medical analysis on the target image; and
upon completion of the medical analysis, outputting at least one of a visual notification signal and an auditory notification signal.

20. The method for extracting medical information of claim 11, wherein
the method further comprises:
outputting a warning signal when at least one of a vertical resolution, a horizontal resolution, and a ratio between the vertical resolution and the horizontal resolution of the target image is less than or equal to a pre-set value.
